# EUROPEAN PATENT APPLICATION

(11) **EP 3 744 245 A1**
(43) Date of publication of application: **02.12.2020**
(21) Application number: 18902889.7
(22) Date of filing: 26.01.2018
(51) Int. Cl.: A61B 5/08, G01S 17/50

(54) **DEVICE AND METHOD FOR MONITORING THE RESPIRATORY RATE OF A SUBJECT**

(71) Applicant: Asociacion Instituto de Biomecanica de Valencia, 46022 Valencia (ES)
(72) Inventor: LAPARRA HERNÁNDEZ, José, 46022 Valencia (ES); IZQUIERDO RIERA, Mateo Dimas, 46022 Valencia (ES); MEDINA RIPOLL, Enrique, 46022 Valencia (ES); PALOMARES OLIVARES, Nicolás, 46022 Valencia (ES); SOLAZ SANAHUJA, José S., 46022 Valencia (ES)
(74) Representative: Capitán García, Maria Nuria
(86) International application number: PCT/ES2018/070060
(87) International publication number: WO 2019/145580

(57) **Abstract**

The invention relates to a device for monitoring the respiratory rate of a subject, which comprises: means for detecting changes in the subject's torso that indicate breathing movements; a processor that receives signals from the detection means and checks that a respiratory frequency of the subject is within pre-established reference respiratory frequency values; and means for preventing emergencies, which are adapted to be activated by the processor. According to the invention, the detection means comprise a laser emitter adapted to project at least one point onto the subject's torso; a camera; and a filter that is coupled to the camera and adapted to detect only the light spectrum emitted by the laser emitter. The application also relates to a method for this purpose.

## Description

### TECHNICAL FIELD OF THE INVENTION

The present invention relates to technologies for the surveillance or monitoring of the respiratory rate of a subject, whether a driver of a vehicle, an athlete, a patient, etc., i.e., a subject involved in any activity that requires maintaining a continued monitoring of their respiratory rate.

Particularly, subject matters of the present application are a device and a method for monitoring the respiratory rate of a subject, based on registering the increases and decreases in volume of the subject's torso when breathing.

### BACKGROUND OF THE INVENTION

Individuals (subjects) usually perform shallow, erratic chest breathing, with normal values at rest between 12 to 20 breaths (inhalation and exhalation) per minute for an adult subject. For example, men typically average between 16 and 18 breaths per minute, while women's average breathing frequency is between 18 and 20 breaths per minute.

Breathing frequency measurement is usually carried out in two ways: by measuring the intake and output of gas with a mask that measures the air volume, usually in a hospital setting; measuring a band attached to the chest as it expands and contracts.

All the activities that a subject carries out influence the frequency of their breathing. While we eat, walk, run, climb a slope, drive a vehicle, and even when we sleep, our body needs to oxygenate itself. The demands, however, are not the same in each activity, so the human body has the incredible ability to adapt to both the environment and the demands of the action being performed.

Likewise, the breathing frequency gives information on the subject's overall health, and specifically, on the state of their lungs and respiratory system. Breathing usually accelerates when the subject has an infection, fever, blood loss or dehydration, lung problems, or other emergencies.

Ventilation and gaseous exchange that occurs when a subject breathes are actions that are maintained during sleep. However, when sleeping, ventilation decreases, since the body does not demand as much energy. The activity of the receiving organs, either of oxygen or carbon dioxide, is less, so the response to variations in these gases is not as effective as when the subject is awake, reducing their breathing frequency.

For this reason, the monitoring of the respiratory frequency or rate of a subject is used in multiple applications, for example, in the control or monitoring of the health condition of hospitalised patients or at home. Also, in the detection of states of stress, drowsiness or negative emotional states in subjects who perform tasks or work that require high concentration, such as air traffic or automated metro controllers.

In the same way, the monitoring of a subject's respiratory frequency or rate can be used to know the state of the occupants of a vehicle, especially its driver, whether a car or any other means of transport with passengers. In this case, breathing monitoring is used to detect possible distractions during driving activity, such as sleep or fatigue, which may lead to unfortunate accidents.

For the monitoring of a subject's respiratory frequency or rate, means that require direct contact with the subject's body are usually used, however, it is not always possible due to the characteristics of the task performed by the subject, or due to the conditions which subject is in.

There are also devices for monitoring the subject's breathing that do not require direct contact with their body, based on the use of volumetric detectors, depth detectors or CO₂ meters, among others, which send the registered values to a data processor (computer), which determines whether the subject's breathing is within predetermined normal breathing values, if not, said data processor activates emergency prevention means.

For example, in patent US 6793242, published on 21 September 2004, a Micropower Impulse Radar (MIR) sensor is used that sends the values it registers to a data processor to determine the breathing frequency of the subject (vehicle driver) based on registering their chest movement. Thus, it is possible to detect whether the driver's breathing is erratic or indicative of a state in which the driver is dozing, or is driving the vehicle in an unsuitable health condition. In the case of confirming that the driver has fallen asleep or health problems that affect driving arise, emergency prevention means are activated, either a visible and/or audible alarm signal, or if the vehicle is equipped with automatic guiding and start-up systems, activating the latter to park and stop the vehicle safely.

However, these known devices are not entirely accurate, especially when the driver is wearing loose clothing or there is some object between the driver's torso and the registering means, leading to misreadings.

For this reason, it is necessary to overcome the aforementioned drawbacks recognised in current technologies for monitoring the respiratory rate of a subject, which, as seen, not only may be applied in the transport sector but may also be useful in other technical sectors, such as the medical equipment industry, aeronautics, high-performance sports, etc.

### DESCRIPTION OF THE INVENTION

The present invention is established and characterised in the independent claims, while the dependent claims describe other characteristics thereof.

An object of the invention is a device for monitoring the respiratory rate of a subject, which comprises:
- means for detecting changes in the subject's torso that indicate breathing movements of the subject,
- a data processor (computer) that receives signals from the detection means about the subject's breathing movements and checks that the subject's respiratory frequency is within pre-established reference respiratory frequency values, and
- emergency prevention means adapted to be actuated by the data processor when the subject's respiratory frequency is outside the pre-established reference respiratory frequency values.

Wherein, the detection means comprise:
- a laser emitter, adapted to project at least one point, preferably a dot matrix or pattern of points, onto the subject's torso,
- a camera, and
- a filter coupled to the camera, adapted to detect only one light spectrum corresponding to that emitted by the laser emitter.

It is checked that the emitter and the camera operate properly in any band of the spectrum, as can be usual in the infrared band, without being limited thereto and therefore being able to be used in any other.

Another object of the invention is a method for monitoring the respiratory rate of a subject, comprising the following steps:
a) projecting at least one point, preferably a dot matrix or pattern of points, onto the subject's torso,
b) capturing images of displacements of the point or points of interest of the pattern on the torso, wherein said displacements can be identified as the subject's breathing movements,
c) determining a respiratory frequency of the subject from the captured images,
d) checking that the subject's respiratory frequency is within pre-established reference respiratory frequency values, and
e) activating emergency prevention means when the subject's respiratory frequency is outside the pre-established reference respiratory frequency values.

In this way, a monitoring of the subject's respiratory frequency or rate can be carried out in an effective and efficient way, without the loose clothing that the subject might be wearing, the presence of any object between the subject's torso and the means used to detect changes in said torso, the subject's own movements (e.g., to pick something up) or external thereto (e.g., the movement of the vehicle in which the subject is located), or the degree of ambient illumination, affecting the result of the measurements on the increases and decreases in the volume of the torso of the subject that allow knowing their actual respiratory frequency and checking if the latter is within pre-established reference respiratory frequency values.

Logically, the pre-established reference respiratory frequency values to be used are selected based on the activity for which the object of the present invention is intended. For example, monitoring the respiratory rate of hospitalised patients, controlling subjects who perform tasks that require high concentration, controlling the training of subjects who practice high-performance sports, etc.

For example, in the case of monitoring a vehicle driver, the pre-established reference respiratory frequency values will correspond to the indicators of a subject at rest who is awake and in an acceptable state of health to drive a vehicle. Thus, the emergency prevention means may be activated when detecting that the subject's actual respiratory frequency is outside such pre-established reference values for a vehicle driver, either because they indicate that he or she is dozing or drowsy, or, quite the opposite, that he or she is in a state of stress or in a negative emotional or health state that prevents them from driving the vehicle with due concentration and safety. Thus avoiding unwanted traffic accidents.

### BRIEF DESCRIPTION OF THE DRAWINGS

This specification is supplemented by a set of drawings illustrating the preferred embodiment and never intended to limit the invention.
Figure 1 represents a schematic view of the device, applied to the monitoring of the respiratory rate of a subject driving a vehicle.
Figure 2 represents a front view of the subject's torso, onto which a dot matrix or pattern of points has been projected.
Figure 3 represents a graph showing a dimensionless oscillation of the increase and decrease in the volume of the subject's torso that corresponds to his or her breathing frequency.

### DETAILED DESCRIPTION OF THE INVENTION

Objects of the present invention are a device for monitoring the respiratory rate of a subject, as well as a method for the same purpose.

The present invention is useful for multiple applications, for example, for monitoring the respiratory rate of hospitalised patients, for controlling subjects who perform tasks that require high concentration, for controlling the training of subjects who practice high-performance sports, among other applications.

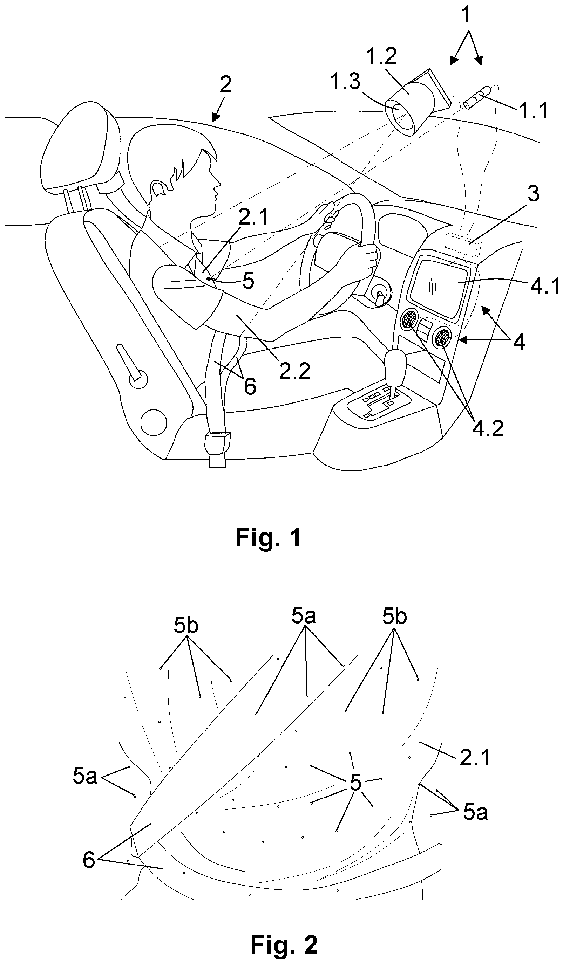

One of the possible examples of embodiment of the invention is shown in Figure 1, in this case, of application in monitoring the respiratory rate of a subject (2) driving a vehicle.

As shown in Figure 1, the device comprises:
- means for detecting (1) changes in a torso (2.1) of the subject (2) that indicate breathing movements of the subject (2),
- a data processor (3), a computer, that receives signals from the detection means (1) about the breathing movements of the subject (2) and checks that a respiratory frequency of the subject (2) is within pre-established reference respiratory frequency values, and
- emergency prevention means (4) adapted to be activated by the processor (3) when the respiratory frequency of the subject (2) is outside the pre-established reference respiratory frequency values.

For their part, the detection means (1) comprise:
- a laser emitter (1.1) adapted to project at least one point (5) onto the torso (2.1) of the subject (2),
- a camera (1.2), and
- a filter (1.3) coupled to the camera (1.2).

Preferably, the laser emitter (1.1) projects a dot matrix or pattern of points (5) onto the torso (2.1) of the subject (2).

As for the filter (1.3), it is adapted to detect only one light spectrum that corresponds to that emitted by the laser emitter (1.1).

Since the laser emitter (1.1) and the filter (1.3) operate at the same wavelength, the degree of ambient illumination is prevented from affecting the retrieval of data captured by the camera (1.2) about the movements experienced by the point(s) (5) projected onto the torso (2.1) of the subject (2) during the latter's breathing. Otherwise, both too bright an ambient light or too weak a light could lead to misreadings of the movements of such points (5).

The advantage of projecting a pattern of points (5) onto the torso (2.1) of the subject (2), see Figure 2, is that if one or several points (5a) of said pattern of points (5) is projected onto an element other than the torso (2.1) of the subject (2) that stands between the latter and the camera (1.2) that captures their movement, for example, an arm (2.2) of the subject (2) or a safety belt (6), or simply, it is projected outside the torso (2.1) of the subject (2), said point(s) (5a) that would lead to the determination of an incorrect breathing frequency of the subject (2) can be discarded, the processor data (3) focusing on points of interest (5b) of said pattern of points (5) that show movements consistent or in accordance with the movement of the torso (2.1) of the subject (2) corresponding to breathing.

Mainly due to aesthetic reasons and to reduce the effect of natural light, it is preferred that the light spectrum in which the laser emitter emits and that the filter detects (1.3) corresponds to a wavelength not visible to the human eye, for example, it could correspond to a wavelength of approximately 800 nm, corresponding to the near infrared. This does not exclude other wavelengths that would be equally viable.

For their part, the emergency prevention means (4) could be an audible and/or visual alarm signal that alerts that the respiratory frequency of the subject (2) is outside the pre-established reference respiratory frequency values. The alarm or alert could be implemented inside the vehicle itself, using a screen (4.1) for the visual signal and/or speakers (4.2) for the audible signal; or, it could be displayed on the screen of a remote computer (not shown in the figures) that is in wireless communication with the device. Or they can act on other types of emergency systems (not shown in the figure), for example, automatic guiding and start-up systems of a vehicle for it to be parked and safely stopped.

There can be multiple emergency prevention means (4) that can be used in the device of the present application, of course, depending on the application for which the latter is intended. For example, if applied to the monitoring of the respiratory rate of a subject (2) being a hospitalised patient, the emergency prevention means (4) could be an audible and/or visual alarm signal generated in the speakers and/or displayed on the computer screen (not shown in the figures) of the nurses' station as appropriate. If it were a respiratory rate monitoring device for a subject (2) applied to a high-performance sports training apparatus (not shown in the figures), the audible and/or visual alarm signal could be generated in the speakers and/or displayed on the training apparatus screen (not shown in the figures) as appropriate.

As for the method also an object of the present application, it is based on projecting at least one point (5) onto a torso (2.1) of the subject (2). In a more preferred embodiment, a dot matrix or pattern of points (5) is projected onto said torso (2.1) of the subject (2).

For example, a laser emitter (1.1) can be used to project the point or points (5) onto the torso (2.1) of the subject (2).

Images of displacements of the point(s) (5) on the torso (2.1) are then captured, wherein such displacements are caused by breathing movements of the subject (2).

For example, for the acquisition or capture of images, a camera (1.2) with a filter (1.3) can be used, wherein the filter (1.3) is adapted to detect only one light spectrum from the laser emitter (1.1).

This image acquisition step starts by capturing a first image where the point(s) of interest (5b) are located, as appropriate, and then, in subsequent captured images, the displacement of the point (s) of interest (5b) on the torso (2.1) of the subject (2) during his or her breathing movements is updated or followed.

A respiratory frequency of the subject (2) is then determined from the captured images.

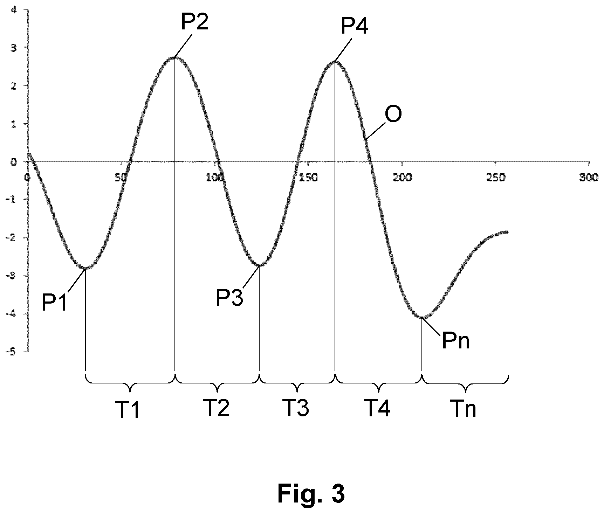

In this step, in the embodiment in which a single point (5) is projected onto the torso (2.1), said point (5) would be a point of interest (5b), its movements are extracted from the captured images, and these movements are integrated throughout the frame of images captured on both sides of a main axis of movement to obtain a dimensionless oscillation (O), seen in Figure 3, which corresponds to the increases and decreases in torso (2.1) volume of the subject (2), to then determine the respiratory frequency of the subject (2) based on said dimensionless oscillation (O).

On the other hand, in the embodiment in which a pattern of points (5) is projected, from the points of interest (5b) followed in the images captured in the preceding step, those which have maintained, throughout these captured images, a displacement consistent with the breathing movement of the subject (2) are determined or selected. Thus, the rest of the points (5) that are not of interest are rejected, for example, the points (5a) that have been projected onto an element (2.2, 6) located between the torso (2.1) of the subject (2) and the camera (1.2), or have been projected outside the torso (2.1) of the subject (2). The movements of the previously selected points of interest (5b) are then extracted from the captured images, and the global or mean movement of said points of interest (5b) is integrated throughout the frame of captured images on both sides of a main axis of movement to obtain the dimensionless oscillation (O) for this case, seen in Figure 3, which corresponds to the increases and decreases in torso (2.1) volume of the subject (2), in order to thus determine the respiratory frequency of the subject (2) based on said dimensionless oscillation (O).

In both embodiments, the respiratory frequency of the subject (2) (breaths per minute (BPM)) can be determined by locating peak values (P1, P2, P3, P4, Pn) in the dimensionless oscillation (O) obtained, which, correspond to the respective maximum and minimum extreme values of the increases and decreases in volume undergone by the torso (2.1) of the subject (2) when breathing, and measuring time values (T1, T2, T3, T4, Tn) that elapse between every two adjacent peak values (P1, P2, P3, P4, Pn), i.e., the times elapsed from peak to peak throughout the dimensionless oscillation (O) obtained. The respiratory frequency of said subject (2) is obtained by calculating the inverse of the mean of said measured time values (T1, T2, T3, T4, Tn).

Once the respiratory frequency of the subject (2) has been determined, it is checked that said respiratory frequency of the subject (2) is within pre-established reference respiratory frequency values; and in the event that said respiratory frequency of the subject (2) is outside the pre-established reference respiratory frequency values, emergency prevention means are activated (4).

Preferably, both the step of determining the respiratory frequency of the subject (2) and of checking that it is within pre-established reference respiratory frequency values are performed by a data processor (3), which will also be the one that commands the activation of the emergency prevention means (4), in case the respiratory frequency of the subject (2) is outside the pre-established reference respiratory frequency values.

As can be understood, these pre-established reference respiratory frequency values are provided to the data processor (3) previously before the method starts. Which values are selected based on the activity for which the object of the present invention is intended. For example, in the case of monitoring a vehicle driver, the pre-established reference respiratory frequency values will correspond to the indicators of a subject at rest who is awake and in an acceptable state of health to drive a vehicle. Thus, the emergency prevention means (4) may be activated when detecting that the actual respiratory frequency of the subject (2) is outside such pre-established reference values for a vehicle driver, either because they indicate that he or she is dozing or drowsy, or, quite the opposite, that he or she is in a state of stress or in a negative emotional or health state that prevents them from driving the vehicle with due concentration and safety. Thus avoiding unwanted traffic accidents.

## Claims

1. Device for monitoring the respiratory rate of a subject, comprising:
- means for detecting (1) changes in a torso (2.1) of the subject (2) that indicate breathing movements of the subject (2),
- a data processor (3) that receives signals from the detection means (1) about the breathing movements of the subject (2) and checks that a respiratory frequency of the subject (2) is within pre-established reference respiratory frequency values, and
- emergency prevention means (4) adapted to be activated by the processor (3) when the respiratory frequency of the subject (2) is outside the pre-established reference respiratory frequency values,
**characterised in that** the detection means (1) comprise a laser emitter (1.1) adapted to project at least one point (5) onto the torso (2.1) of the subject (2), a camera (1.2) and a filter (1.3) coupled to the camera (1.2), wherein the filter (1.3) is adapted to detect only one light spectrum that corresponds to that emitted by the laser emitter (1.1).

2. Device according to claim 1, wherein the laser emitter (1.1) projects a dot matrix or pattern of points (5) onto the torso (2.1) of the subject (2).

3. Device according to Claims 1 or 2, wherein the light spectrum emitted by the laser emitter and detected by the filter (1.3) corresponds to a wavelength not visible to the human eye.

4. Device according to Claim 3, wherein the light spectrum corresponds to a wavelength of approximately 800 nm.

5. Device according to Claim 1, wherein the emergency prevention means (4) are a sound or visual alarm signal.

6. Device according to Claim 1, wherein the emergency prevention means (4) are means that act on automatic guiding and start-up systems of a vehicle for it to be parked and stopped.

7. Method for monitoring the respiratory rate of a subject, comprising the following steps:
a) projecting at least one point (5) onto a torso (2.1) of the subject (2),
b) capturing images of displacements of the point (5) on the torso (2.1), wherein said displacements are caused by breathing movements of the subject (2),
c) determining a respiratory frequency of the subject (2) from the captured images,
d) checking that the respiratory frequency of the subject (2) is within pre-established reference respiratory frequency values,
e) activating emergency prevention means (4) when the respiratory frequency of the subject (2) is outside the pre-established reference respiratory frequency values.

8. Method according to Claim 7, wherein, in step a), a laser emitter (1.1) is used.

10. Method according to Claim 8, wherein, in step b), a camera (1.2) with a filter (1.3) is used, wherein the filter (1.3) is adapted to detect only one light spectrum from the emitter laser (1.1).

11. Method according to claim 7, wherein step c) comprises the following sub-steps:
c1.1) extracting the movements of point (5) from the captured images,
c1.2) integrating the movements of point (5) to obtain a dimensionless oscillation (O) that corresponds to increases and decreases in a volume of the torso (2.1), and
c1.3) determining the respiratory frequency of the subject (2) based on the dimensionless oscillation (O).

12. Method according to Claim 7, wherein, in step a), a dot matrix or pattern of points (5) is projected onto the torso (2.1) of the subject (2).

13. Method according to Claim 12, wherein step c) comprises the following sub-steps:
c2.1) selecting points of interest (5b) from the pattern of points (5) that develop displacements consistent with the breathing movement of the subject (2),
c2.2) extracting the movements of the previously selected points of interest (5b) from the captured images,
c2.3) integrating the global or mean movement of said points of interest (5b) to obtain a dimensionless oscillation (O) that corresponds to increases and decreases in a volume of the torso (2.1), and
c2.4) determining the respiratory frequency of the subject (2) based on the dimensionless oscillation (O).

14. Method according to Claims 11 or 13, wherein, in sub-steps c1.3) or c2.4), the respiratory frequency of the subject (2) is determined by locating peak values (P1, P2, P3, P4, Pn) in the dimensionless oscillation (O) obtained in the preceding sub-step, by measuring time values (T1, T2, T3, T4, Tn) that elapse between every two adjacent peak values (P1, P2, P3, P4, Pn), and by calculating the inverse of the mean of said measured time values (T1, T2, T3, T4, Tn).

15. Method according to Claim 7, wherein steps c) and d) are performed by a data processor (3).

16. Method according to Claim 14, wherein, in step e), the emergency prevention means (4) are activated by the data processor (3).

## Amended claims

### Amended claims under Art. 19.1 PCT

1. Device for monitoring the respiratory rate of a subject, comprising:
- means for detecting (1) changes in a torso (2.1) of the subject (2) that indicate breathing movements of the subject (2),
- a data processor (3) that receives signals from the detection means (1) about the breathing movements of the subject (2) and checks that a respiratory frequency of the subject (2) is within pre-established reference respiratory frequency values, and
- emergency prevention means (4) adapted to be activated by the processor (3) when the respiratory frequency of the subject (2) is outside the pre-established reference respiratory frequency values,
wherein, the detection means (1) comprise a laser emitter (1.1), a camera (1.2) and a filter (1.3) coupled to the camera (1.2), **characterised in that** the laser emitter (1.1) is adapted to project a dot matrix or pattern of points (5) onto the torso (2.1) of the subject (2), the camera (1.2) is adapted to capture images of displacements of the points (5) on the torso (2.1) of the subject (2), and the filter (1.3) is adapted to detect only one light spectrum that corresponds to that emitted by the laser emitter (1.1).

2. Device according to Claim 1, wherein the light spectrum emitted by the laser emitter and detected by the filter (1.3) corresponds to a wavelength not visible to the human eye.

3. Device according to Claim 2, wherein the light spectrum corresponds to a wavelength of approximately 800 nm.

4. Device according to Claim 1, wherein the emergency prevention means (4) are a sound or visual alarm signal.

5. Device according to Claim 1, wherein the emergency prevention means (4) are means that act on automatic guiding and start-up systems of a vehicle for it to be parked and stopped.

6. Method for monitoring the respiratory rate of a subject, comprising the following steps:
a) projecting a dot matrix or pattern of points (5) onto a torso (2.1) of the subject (2),
b) capturing images of displacements of the points (5) on the torso (2.1), wherein said displacements are caused by breathing movements of the subject (2),
c) determining a respiratory frequency of the subject (2) from the captured images,
d) checking that the respiratory frequency of the subject (2) is within pre-established reference respiratory frequency values,
e) activating emergency prevention means (4) when the respiratory frequency of the subject (2) is outside the pre-established reference respiratory frequency values.

7. Method according to Claim 6, wherein, in step a), a laser emitter (1.1) is used.

8. Method according to Claim 7, wherein, in step b), a camera (1.2) with a filter (1.3) is used, wherein the filter (1.3) is designed to detect only one light spectrum from the emitter laser (1.1).

9. Method according to Claim 6, wherein step c) comprises the following sub-steps:
c1.1) selecting points of interest (5b) from the pattern of points (5) that develop displacements consistent with the breathing movement of the subject (2),
c1.2) extracting the movements of the previously selected points of interest (5b) from the captured images,
c1.3) integrating the global or mean movement of said points of interest (5b) to obtain a dimensionless oscillation (O) that corresponds to increases and decreases in a volume of the torso (2.1), and
c1.4) determining the respiratory frequency of the subject (2) based on the dimensionless oscillation (O).

10. Method according to Claim 9, wherein, in sub-step c1.4), the respiratory frequency of the subject (2) is determined by locating peak values (P1, P2, P3, P4, Pn) in the dimensionless oscillation (O) obtained in the preceding sub-step, by measuring time values (T1, T2, T3, T4, Tn) that elapse between every two adjacent peak values (P1, P2, P3, P4, Pn), and by calculating the inverse of the mean of said measured time values (T1, T2, T3, T4, Tn).

11. Method according to Claim 6, wherein steps c) and d) are performed by a data processor (3).

12. Method according to Claim 11, wherein, in step e), the emergency prevention means (4) are activated by the data processor (3).

Statement under Art. 19.1 PCT
Differences between the claims as filed and the claims as amended:
- Claim 1 has been modified including the content of the original claim 2 and the content of lines 25-30, page 10 of the Description.
- Claim 2 has been deleted.
- Claim 3 is now claim 2 and depends on the new claim 1.
- Claim 4 is now claim 3 and depends on the new claim 2.
- Claim 5 is now claim 4.
- Claim 6 is now claim 5.
- Claim 7 is now claim 6 and it has been modified including the content of the original claim 12.
- Claim 8 is now claim 7 and depends on the new claim 6.
- Claim 9, by obvious mistake, was not included in the original set of claims.
- Claim 10 is now claim 8 and depends on the new claim 7.
- Claim 11 has been deleted.
- Claim 12 has been deleted.
- Claim 13 is now claim 9 and depends on the new claim 6.
- Claim 14 is now claim 10 and depends on the new claim 9.
- Claim 15 is now claim 11 and depends on the new claim 6.
- Claim 16 is now claim 12 and depends on the new claim 11.

Here below are enclosed the claims as amended where modifications of claims as filed can be followed as:
- text deleted as filed is crossed out,
- text added as amended is underlined.
